# EUROPEAN PATENT APPLICATION

(11) **EP 4 656 167 A1**
(43) Date of publication of application: **03.12.2025**
(21) Application number: 25178951.7
(22) Date of filing: 27.05.2025
(51) Int. Cl.: A61F 5/56

(54) **METHOD FOR PROVIDING A NEUROMUSCULAR ORTHOTIC DEVICE AND NEUROMUSCULAR ORTHOTIC DEVICE**

(30) Priority: 28.05.2024 IT 202400012025
(71) Applicant: LaPonte, Rosa Maria, 20831 Seregno MB (IT); Sergi, Gianandrea, 20155 Milano (IT)
(72) Inventor: LaPonte, Rosa Maria, 20831 Seregno MB (IT); Sergi, Gianandrea, 20155 Milano (IT)
(74) Representative: Modiano, Micaela Nadia

(57) **Abstract**

A method for providing a neuromuscular orthotic device (1) which comprises a contoured dental plate to be fitted over the lower arch of a patient and has a contact surface with the upper arch of the patient which has different tooth shapes from those of the patient, in order to modify for a number of hours over the day, or at night, the relationship between the upper and lower arches, so determining a new dental occlusion which enables an adequate postural contact between mandible and maxilla in relation to the cranium.

In this manner, in addition to providing mandibular stability at rest, the device enables a cranial-mandibular repositioning, which guides the patient to the acquisition of a new and correct dental occlusion and a new neuro-motor engram, rebalancing the stomatognathic system and overall posture.

## Description

The present invention relates to a method for providing a neuromuscular orthotic device and the device obtained with such method.

In the orthodontics sector the use is known of occlusal devices, more commonly called "bite plates", which are used in the treatment of some problems in the stomatognathic apparatus: grinding and clenching of teeth, alterations of the dental occlusion, chronic snoring, and sleep apnea.

More specifically, a bite plate is an appliance, typically made of acrylic resin, which is placed between the two dental arches for a period of time that varies based on the underlying disorder.

The bite plate is contoured to fit over the dental arch on which it is applied, and it is made in the laboratory after taking a dental impression in a mold.

In any case, conventional bite plates are not devoid of drawbacks, among which is the fact that they provide mandibular stability only at rest, without a cranial-mandibular repositioning, which would guide the patient to the acquisition of a new and correct dental occlusion and a new neuro-motor engram, rebalancing the stomatognathic system and overall posture.

Furthermore, conventional bite plates, differently from other orthodontic appliances, are static devices that are not designed to adapt to the new occlusal dynamics that the patient can present over time during its use.

The aim of the present invention consists in devising a method for providing a neuromuscular orthotic device that is capable of leading to a cranial-mandibular repositioning, with the consequent acquisition by the patient of a new and correct dental occlusion and of a new neuro-motor engram, rebalancing the stomatognathic system and overall posture, as well as to mandibular stability at rest.

Within this aim, an object of the present invention is to provide a neuromuscular orthotic device that is capable of overcoming the limitations and drawbacks of conventional bite plates.

Another object of the present invention consists in devising a method for providing a neuromuscular orthotic device and providing a neuromuscular orthotic device that are capable of offering the widest assurances of reliability, efficiency and operation.

This aim and this and other objects which will become more evident hereinafter are achieved by a method for providing a neuromuscular orthotic device according to claim 1 and by a neuromuscular orthotic device according to claim 8.

Further characteristics and advantages of the invention will become more apparent from the detailed description of a preferred, but not exclusive, embodiment of a method for providing a neuromuscular orthotic device, illustrated by way of non-limiting example with the aid of the accompanying drawings wherein:

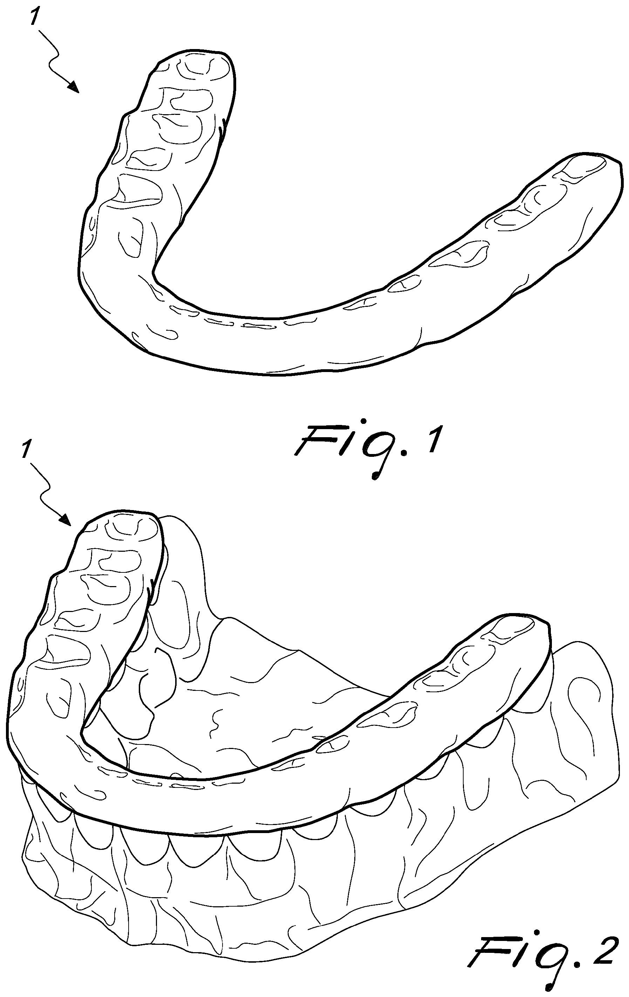

- Figure 1 is a perspective view of a neuromuscular orthotic device according to the invention;

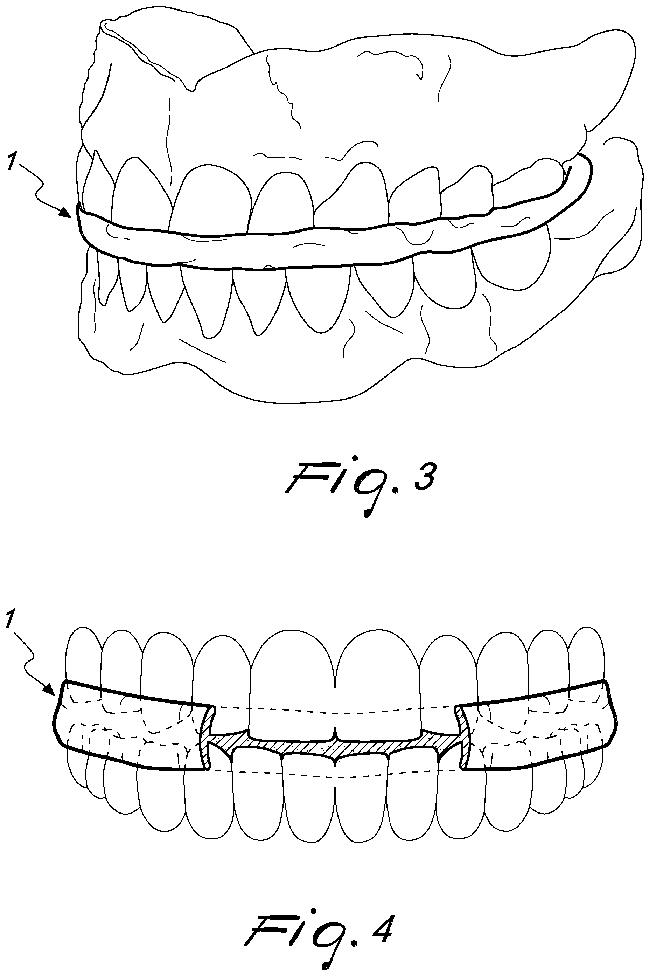

- Figures 2 and 3 are two perspective views of the orthotic device shown in Figure 1 applied on a lower arch of a patient, without and with the upper arch;
- Figure 4 is a front elevation view of the orthotic device shown in Figure 3.

With reference to the figures, the method for providing a neuromuscular orthotic device, generally designated by the reference numeral 1, comprises a first step of clinical evaluation and clinical instrument-based tests of the patient.

In this first step, of clinical evaluation and instrument-based tests, wherein the doctor analyzes the condition of the patient, at least one and preferably all of the tests comprised in the following group are conducted: extra- and intra-oral evaluation of the patient's dental arches, ortho-panoramic (OPT), antero-posterior (AP) and latero-lateral (L-L) teleradiograph, stabilometric platform and postural examination, surface electromyography (EMG) and kinesiography, maxillofacial CT and transcutaneous electrical nerve stimulation (TENS).

Subsequently, the method proceeds with the taking of dental impressions, preferably alginate and wax, in maximum intercuspation of the present occlusal state to obtain two upper models and two lower models.

Alternatively, the impressions can be performed by means of three-dimensional scanning.

The alginate impression is cast for the first time with extra-hard type IV plaster, and for the second time with orthodontic plaster for models.

At this point the method proceeds with an essential step of the method according to the invention, i.e. a step of induced muscle relaxation of the patient's masticatory muscular district.

This step of induced muscle relaxation comprises, advantageously, trigeminal transcutaneous electrical nerve stimulation (TENS).

Interruption of the occlusal contacts during application of TENS has a major effect, not only locally on the masticatory muscles, but also on the central nervous system.

At this level, in fact, we see the cancellation, or a great reduction, of the signals from the periodontal tissues which are responsible for our space/time awareness of the exact mandibular position in space.

The application of TENS, therefore, interrupts the constant development of neuro-motor engrams that originate from occlusal contact, reducing muscle tone and increasing the physical length of the muscle fibers, until the physiological position of rest is reached.

This position, not conditioned by occlusion, is the ideal muscular rest position, in which all the functions of the stomatognathic apparatus are more efficient in energy terms.

But the habitual rest position of the mandible is an adaptation to the present occlusal condition and to cranial-cervical posture, although it is not always the most functional for the stomatognathic system.

Subsequently, the method proceeds with a step of registration of the present occlusal state, which is performed with an orientable and transferable support system for dental impressions (S.I.D.-O.T.), and in an articulator (Artex), according to a custom individual horizontal plane.

At this point the method proceeds with a step of construction of a foundation by means of the wax dental impressions obtained from the step of taking dental impressions.

In more detail, the foundation is constructed by means of an ideal horizontal plane, customized to each patient and passing through three points: the two tuber maxillae and the retroincisal papilla.

Then the method proceeds to an additional step of construction of a new custom wax-up, configured according to the data acquired in the step of clinical evaluation and instrument-based tests.

The outcome of the kinesiography and mandibular tests, EMG and trigeminal TENS is therefore indispensable to the registration of dental occlusion in maximum intercuspation (myocentric occlusion), in order to determine the dysfunctional parameters to be corrected and configure the orthotic device based on the data obtained.

The method then proceeds to a step of positioning one of the upper models and one of the lower models, obtained from the step of taking dental impressions, in an individual articulator, interposing the new custom wax-up and taking advantage of the occlusal registration fork obtained from the step of registration of the present occlusal state.

At this point it is possible to proceed with a step of extraction of the models with the new custom wax-up interposed from the individual articulator and positioning thereof in a venticulator (muffle), with fixation thereof using type II plaster.

Finally, the method proceeds with a step of removal of the new custom wax-up from the venticulator and with the casting of resin into the venticulator to obtain a rough neuromuscular orthotic device 1.

Then the method proceeds with a step of finishing by stock-removal machining of the rough neuromuscular orthotic device to obtain a finished neuromuscular orthotic device 1.

Subsequently to the step of finishing, the method can proceed with the following steps:
- extraction of the models from the venticulator;
- positioning of the models in the individual articulator;
- verification of the occlusal contacts configured on the basis of the data previously obtained with the step of clinical evaluation and instrument-based tests.

Also subsequently to the step of finishing, the method can proceed with the repositioning of the finished neuromuscular orthotic device 1 on the model obtained by foundation forming, to verify the occlusal contacts by means of articulating papers and checking the match with those of the articulator.

The neuromuscular orthotic device 1 is then ready to be handed to the patient.

In more detail, the neuromuscular orthotic device 1 thus obtained therefore comprises a dental plate made of resin, shaped to be fitted on the lower arch of a patient and having a contact surface with the upper arch of the patient which has different tooth shapes from those of the patient.

During the treatment, the neuromuscular orthotic device 1 is progressively modified over time, until the patient is guided to a new physiological dental occlusion and to a correct cranial-cervical posture.

Then periodic (monthly) checks must be performed to monitor, through the use of papers, the changes in the occlusal contacts as a consequence of the neuromuscular adaptations that have taken place in the patient wearing the neuromuscular orthotic device 1, and to "remodel" said device based on what is shown on these papers, until such time as the ideal occlusal contacts are reached, or the most functional contacts for that specific patient.

The above treatment can have an average duration of 24 months, at the end of which time the central nervous system will have fully acquired a new neuromuscular engram, necessary for generating a new occlusal configuration and maintaining it over time.

Prior to concluding the treatment, the new occlusal stability is verified through TENS, EMG and kinesiography and, if stability is reached, the neuromuscular orthotic device 1 will not be modified further over time.

In practice it has been found that the method and the device, according to the present invention, achieve the set aim and objects.

In fact, the neuromuscular orthotic device according to the invention treats several problems of the stomatognathic system by acting on muscles, joints and occlusion.

Restoring a physiological occlusion ensures stability of the mandibular resting position and improved neuromotor efficiency of the stomatognathic system in terms of muscular balancing during mastication, deglutition and phonation.

The principal problems that can be treated by the neuromuscular orthotic device according to the invention are: grinding and clenching of teeth, alterations in dental occlusion, dysfunctions in the temporomandibular joint, muscular-tension headaches, post-traumatic cranial-cervical-mandibular pains and disorders, dizziness and balance disorders, tinnitus, cranial-cervical postural alterations, and scoliosis.

Another advantage of the neuromuscular orthotic device according to the invention consists in that this can play a particularly important role for sports practitioners in overall neuromuscular rebalancing.

In fact, although the device according to the invention presents a configuration that, on the face of it, is similar to that of a conventional bite plate, by virtue of the method used to provide it the device according to the invention assumes a neuromuscular orthotic functionality that makes it possible to rebalance the function of the stomatognathic system.

It is, in fact, a plate for the lower dental arch that, differently from a conventional bite plate, presents a contact surface with the upper arch that has different tooth shapes from those present.

This results in a modification, for a number of hours over the day, or at night, of the relationship between the upper arch and the lower arch, so determining a new dental occlusion which enables an adequate postural contact between mandible and maxilla in relation to the cranium.

Therefore, in addition to obtaining mandibular stability at rest, it is possible to thus obtain a cranial-mandibular repositioning, which guides the patient to the acquisition of a new and correct dental occlusion and a new neuro-motor engram, rebalancing the stomatognathic system and overall posture.

Furthermore, by virtue of the fact that the device according to the invention is constructed according to the Jankelson principles of neuromuscular orthodontics, which consider dental occlusion as a dynamic aspect of the stomatognathic system, rather than a static characteristic, the device according to the invention enables a better treatment of cranial-facial disorders, because it treats the underlying cause of the alterations in the occlusion, so restoring a physiological neuromuscular equilibrium.

In fact, determining the physiological mandibular resting position is the central focus of the method according to the invention that precedes the construction of the device.

This position is defined after relaxing and deconditioning the masticatory muscles by means of TENS, placing the attention on the neuromuscular status of the stomatognathic system as the foundation for the construction of a new physiological occlusion.

When the mandible is in the inactive position, there is an interocclusal space between the dental arches that is three-dimensional and varies based on muscle tone.

During every act of swallowing, the muscular contraction status is modified, adapting to an occlusal position in maximum intercuspation (a mandibular position in which the cusps of the teeth of both arches are fully interposed with each other).

This latter aspect plays a crucial role, because the occlusal dental contacts send stimuli to the central nervous system, which induce the patient to assume a cranial-cervical-mandibular resting position that is functional and convenient for the mandibular closure movement in maximum intercuspation.

In the presence of alterations of dental occlusion, the habitual resting position will be incongruous and dysfunctional.

The device according to the invention acts precisely in this manner to rebalance the physiological function of the stomatognathic system.

Worn at night, when many involuntary acts of deglutition occur, the device according to the invention sends new stimuli to the central nervous system, determined on the basis of the data obtained from the preliminary evaluation, to "reprogram" the rest configuration of the mandible.

This stimulation, repeated over time, guides the patient to assume a physiological dental occlusion and to acquire a new, more functional neuromotor engram.

The method and the device, thus conceived, are susceptible of numerous modifications and variations, all of which are within the scope of the appended claims.

Moreover, all the details may be substituted by other, technically equivalent elements.

In practice the materials employed, provided they are compatible with the specific use, and the contingent dimensions and shapes, may be any according to requirements and to the state of the art.

The disclosures in Italian Patent Application No. 102024000012025 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A method for providing a neuromuscular orthotic device, **characterized in that** it comprises the following steps:
- clinical evaluation of the patient and instrument-based tests;
- taking alginate and wax dental impressions in maximum intercuspation of the present occlusal state to obtain two upper models and two lower models;
- induced muscle relaxation of the patient's masticatory muscular district;
- registration of the present occlusal state;
- construction of a foundation by means of the wax dental impressions obtained from said step of taking dental impressions;
- construction of a new custom wax-up, configured according to the data acquired in said step of clinical evaluation and instrument-based tests;
- positioning of one of said upper models and one of said lower models in an individual articulator, interposing said new custom wax-up and taking advantage of the occlusal registration fork obtained from said step of registration of the present occlusal state;
- extraction of said models with said new custom wax-up interposed from said individual articulator and positioning thereof in a venticulator (muffle), with fixation thereof;
- removal of said new custom wax-up from said venticulator and casting of resin into said venticulator to obtain a rough neuromuscular orthotic device;
- finishing by stock-removal machining of said rough neuromuscular orthotic device to obtain a finished neuromuscular orthotic device (1).

2. The method according to claim 1, **characterized in that** said step of clinical evaluation and instrument-based tests comprises at least one of the tests comprised in the group constituted by: extra- and intra-oral evaluation of the patient's dental arches, ortho-panoramic, antero-posterior and latero-lateral teleradiograph, stabilometric platform and postural examination, surface electromyography and kinesiography, maxillofacial CT and transcutaneous electrical nerve stimulation (TENS).

3. The method according to claim 1 or 2, **characterized in that** said step of induced muscle relaxation comprises trigeminal transcutaneous electrical nerve stimulation.

4. The method according to one or more of the preceding claims, **characterized in that** said step of registration of the present occlusal state is performed with an orientable dental impression support system and in an articulator according to a customized individual horizontal plane.

5. The method according to one or more of the preceding claims, **characterized in that** in said step of construction, said foundation is constructed by means of an ideal horizontal plane, customized to each patient and passing through three points: the two tuber maxillae and the retroincisal papilla.

6. The method according to one or more of the preceding claims, **characterized in that** it comprises, after said step of finishing, the following steps:
- extraction of said models from said venticulator;
- positioning of said models in said individual articulator;
- verification, in which the occlusal contacts configured on the basis of the data previously obtained with said step of clinical evaluation and instrument-based tests are verified.

7. The method according to one or more of the preceding claims, **characterized in that** it comprises, after said step of finishing, the repositioning of said finished neuromuscular orthotic device on the model obtained by foundation forming, to verify the occlusal contacts by means of articulating papers and checking the match with those of the articulator.

8. A neuromuscular orthotic device (1) obtained by means of a method according to one or more of the preceding claims.

9. The neuromuscular orthotic device (1) according to claim 8, **characterized in that** it comprises a dental plate (X) shaped to be fitted on the lower arch of a patient and having a contact surface with the upper arch of said patient which has different tooth shapes from those of said patient.
